# EUROPEAN PATENT APPLICATION

(11) **EP 1 990 063 A1**
(43) Date of publication of application: **12.11.2008**
(21) Application number: 07714959.9
(22) Date of filing: 27.02.2007
(51) Int. Cl.: A61K 49/00, C07D 493/10

(54) **TUMOR-SELECTIVE FLUORESCENT DYE**

(30) Priority: 28.02.2006 JP 2006051533
(71) Applicant: The University of Tokyo, Bunkyo-ku, Tokyo 113-8654 (JP)
(72) Inventor: NAGANO, Tetsuo, Tokyo 113-8654 (JP); URANO, Yasuteru, Tokyo 113-8654 (JP); KOJIMA, Hirotatsu, Tokyo 113-8654 (JP); FUJIKAWA, Yuuta, Tokyo 113-8654 (JP); TAKAMATSU, Tetsuro, Kyoto-shi, Kyoto 603-8802 (JP); HARADA, Yoshinori, Kyoto-shi, Kyoto 606-0817 (JP)
(74) Representative: Wibbelmann, Jobst
(86) International application number: PCT/JP2007/053566
(87) International publication number: WO 2007/099924

(57) **Abstract**

[Object] To provide a staining agent that is capable of achieving selective fluorescent staining of a tumor cell or a tumor tissue.

[Means to Achieve the Object] A tumor cell or tumor tissue-selective fluorescent staining agent comprising a compound represented by the following general formula (I): 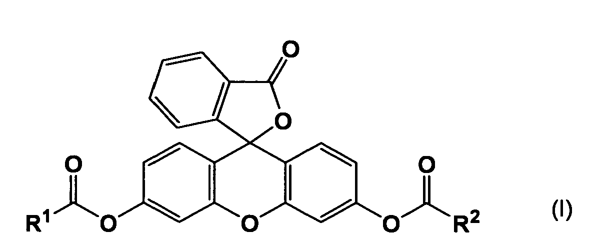 wherein R¹ and R² independently represent a C₁-C₄ alkyl group which may be substituted, a C₂-C₄ alkenyl group which may be substituted, a C₂-C₄ alkynyl group which may be substituted, an aryl group which may be substituted, or a heteroaryl group which may be substituted.

## Description

### Technical Field

The present invention relates to a tumor selective fluorescent staining agent. More specifically, the present invention relates to a staining agent that is capable of achieving selective fluorescent staining of tumor cells or tumor tissues, and is used for selectively imaging tumor cells or tumor tissues in endoscopic cancer diagnosis, and the like.

### Background Art

Optical diagnoses have recently been highly focused as a powerful means for detecting a pathological lesion close to a surface layer with high sensitivity, and an example thereof includes cancer diagnosis by fluorescence endoscopy. 5-Aminolevulinic acid (5-ALA) and porphyrin are used at present for staining tumor tissues in endoscopic diagnosis. However, methods using these staining agents have problems that the methods need prolonged time before the staining agents are accumulated in the tumor tissues, and the like. Accordingly, for detection of cancer, particularly in endoscopic diagnosis and the like, it has been desired to provide a means for staining tumor tissues selectively to distinguish the cells from normal cells.

Fluorescein ester derivatives, such as fluorescein diacetate (FDA) as a fluorescein derivative, are known (see, for example, Non-patent document 1 and the like). These ester derivatives produce strongly fluorescent fluoresceins when the ester moieties are hydrolyzed. The ester derivatives such as FDA are liposoluble because of the presence of an ester group, and therefore they have property of easy permeability through cell membrane. Whilst, fluorescein, which is produced intracellularly through hydrolysis by an esterase in the cell, is a hydrophilic substance, and thus has a property hard to permeate through cell membrane so as to be retained inside the cell.

By utilizing these properties, a technique for measuring an intracellular esterase (technique of utilizing FDA as an esterase probe), and a technique for fluorescently staining cells have been developed, in which FDA is allowed to be uptaken into cells and fluorescence emitted from fluorescein produced through hydrolysis by an intracellular esterase is measured (Non-patent document 2). Although FDA has been used as an esterase probe or a staining agent for uniformly staining tissues as described above, no method is known where the substance is used to distinguish specific cells such as tumor cells from normal cells and selectively staining said cells. Further, no method is known where FDA is used as a contrast medium for endoscopic diagnosis.
Non-patent document 1: Proc. Natl. Acad. Sci. USA, 55, 134-141, 1966
Non-patent document 2: The Journal of Histochemistry and Cytochemistry, 29, pp.503-510, 1981

### Disclosure of the Invention

### Object to be Achieved by the Invention

An object of the present invention is to provide a staining agent that is capable of selectively staining tumor cells or tumor tissues with fluorescence. More specifically, the object of the present invention is to provide a staining agent that has the aforementioned feature, and is useful as a fluorescent contrast medium for endoscopy for selectively staining tumor tissues with fluorescence in endoscopic diagnosis and the like.

### Means for Achieving the Object

The inventors of the present invention conducted various researches to achieve the foregoing object. As a result, they found that by using fluorescein ester derivatives, such as FDA conventionally used as esterase probes or the like, tumor cells and tumor tissues were successfully stained selectively with fluorescence, and further found that these ester derivatives had superior characteristics as contrast media for endoscopy. The present invention was accomplished on the basis of the aforementioned findings.

The present invention thus provides a tumor cell or tumor tissue-selective fluorescent staining agent comprising a compound represented by the following general formula (I): 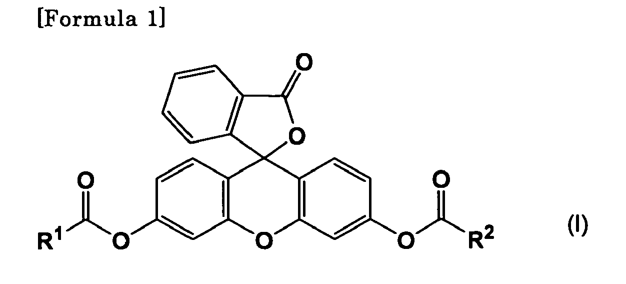 wherein R¹ and R² independently represent a C₁-C₄ alkyl group which may be substituted, a C₂-C₄ alkenyl group which may be substituted, a C₂-C₄ alkynyl group which may be substituted, an aryl group which may be substituted, or a heteroaryl group which may be substituted.

According to preferred embodiments of the aforementioned invention, provided are the aforementioned fluorescent staining agent, wherein R¹ and R² independently represent a C₁-C₄ alkyl group, a C₂-C₄ alkenyl group, an aryl group, or a heteroaryl group; the aforementioned fluorescent staining agent, wherein R¹ and R² independently represent a C₁-C₄ alkyl group, or a C₂-C₄ alkenyl group; the aforementioned fluorescent staining agent, wherein R¹ and R² independently represent a C₁-C₃ alkyl group, or a C₂-C₃ alkenyl group; and the aforementioned fluorescent staining agent, wherein R¹ and R² represent -CH=CH₂.

From another aspect of the present invention, also provided are a diagnostic agent comprising the aforementioned fluorescent staining agent as an active ingredient, and a tumor tissue selective contrast medium comprising the aforementioned fluorescent staining agent as an active ingredient. The aforementioned contrast medium is useful for endoscopic cancer diagnosis, in particular, cancer diagnosis using a fluorescence endoscope that can emit an excitation light having an excitation wavelength optimal for fluorescein (for example, excitation light of about 488 nm) as a light source of the endoscope.

From another aspect, the present invention further provides use of a compound represented by the aforementioned general formula (I) for the manufacture of the aforementioned fluorescent staining agent, diagnostic agent, or contrast medium; a method for staining a tumor cell or tumor tissue, which comprises the step of bringing a compound represented by the aforementioned general formula (I) into contact with the tumor cell or tumor tissue; a method for detecting a tumor cell or tumor tissue, which comprises the steps of bringing a compound represented by the aforementioned general formula (I) into contact with the tumor cell or tumor tissue, then irradiating the cell or tissue with an excitation light for fluorescein, and measuring fluorescence emitted from fluorescein; and a method for imaging a tumor tissue, which comprises the steps of bringing a compound represented by the aforementioned general formula (I) into contact with the tumor cell or tumor tissue, then irradiating the cell or tissue with an excitation light for fluorescein, and measuring fluorescence emitted from fluorescein.

The present invention also provides a method for diagnosing a cancer, which comprises the steps of bringing a compound represented by the aforementioned general formula (I) into contact with a diagnosis site, then irradiating the diagnosis site with an excitation light for fluorescein, and measuring fluorescence emitted from fluorescein to determine the presence or absence of a tumor tissue; and a method for diagnosing a cancer using an endoscope, which comprises the steps of bringing a compound represented by the aforementioned general formula (I) into contact with a diagnosis site, then irradiating the diagnosis site with an excitation light for fluorescein from a light source of an endoscope, and detecting fluorescence emitted from fluorescein by using the endoscope to determine the presence or absence of a tumor tissue.
Further, a cyanine compound may be used with or instead of the compound represented by the aforementioned general formula (I) in the aforementioned diagnostic agent, contrast medium and diagnosis method, and such diagnostic agent, contrast medium and diagnosis method are also provided by the present invention.

### Brief Description of the Drawings

[Fig. 1] This figure shows changes with passage of time in fluorescence intensity ratios of the candidate compounds mentioned in Example 1.
[Fig. 2] This figure shows changes with passage of time in fluorescence intensity ratios of fluorescein ester derivatives.
[Fig. 3] This figure is a schematic view of an experimental system using an endoscope.
[Fig. 4] This figure depicts photographs showing results of endoscopic observation of rat esophagus cancer. In the photographs, (a) shows a white light image, and (b) shows a fluorescent image.
[Fig. 5] This figure depicts photographs showing results of endoscopic observation of rat colon cancer. In the photographs, FL shows a fluorescent image under blue excitation light, and WL shows a white light image.

### Best Mode for Carrying out the Invention

In the general formula (I), the C₁-C₄ alkyl group may be a straight, branched or cyclic alkyl group or an alkyl group consisting of a combination thereof, and examples include, for example, methyl group, ethyl group, n-propyl group, isopropyl group, cyclopropyl group, n-butyl group, isobutyl group, sec-butyl group, tert-butyl group, cyclopropylmethyl group, and the like. Among them, a C₁-C₃ alkyl group is preferred. The C₂-C₄ alkenyl group may be a straight, branched or cyclic alkenyl group, or an alkenyl group consisting of a combination thereof, and may contain one or two double bonds. Examples of the alkenyl group include, for example, vinyl group, n-propenyl group, isopropenyl group, n-butenyl group, and the like. The C₂-C₄ alkynyl group may be a straight, branched or cyclic alkynyl group, or an alkynyl group consisting of a combination thereof, and may contain one or two triple bonds, or one triple bond and one double bond. Examples of the alkynyl group include, for example, ethynyl group, n-propynyl group, n-butynyl group, and the like.

In the general formula (I), as the aryl group, a monocyclic or condensed polycyclic aromatic hydrocarbon group can be used, and examples thereof include phenyl group, naphthyl group, and the like. As the heteroaryl group, a monocyclic or condensed polycyclic aromatic group containing one or more hetero atoms as ring constituting atoms can be used. Examples of the hetero atom include nitrogen atom, oxygen atom, sulfur atom, and the like. When two or more hetero atoms are contained, they may be the same or different. More specific examples include furyl group, thienyl group, pyrrole group, pyridyl group, imidazolyl group, pyrimidyl group, and the like.

When the terminology "may be substituted" is used for a functional group in the present specification, type, number and substitution position of substituent are not particularly limited. Examples of the substituent include, for example, hydroxyl group, amino group, carboxyl group, oxo group, an alkoxycarbonyl group, a halogen atom (examples of the halogen atom include fluorine atom, chlorine atom, bromine atom, iodine atom and the like), but the substituent is not limited to these examples. R¹ and R² may be the same or different, and R¹ and R² are preferably the same from a synthetic viewpoint.

Typical compounds represented by the general formula (I) include the compound in which R¹ and R² represent methyl group (fluorescein diacetate, FDA), compound in which R¹ and R² represent vinyl group (fluorescein diacrylate, FDAcr), compound in which R¹ and R² represent ethyl group (FDP), compound in which R¹ and R² represent n-propyl group (FDB), compound in which R¹ and R² represent n-butyl group (FDC), compound in which R¹ and R² represent phenyl group (FDBz), compound in which R¹ and R² represent 2-furyl group (FDFu), and the like, but not limited to these examples.

The typical compounds represented by the general formula (I) are all known compounds, and listed in, for example, the catalogues of reagents produced by Molecular Probes and Aldrich, and they can be readily obtained by those skilled in the art. Further, they can also be easily prepared according to the production methods of the known compounds. For example, fluorescein diesters including FDAcr are described in Rotoman, B. et al., Proc. Natl. Acad. Sci. USA, 55, 134-141, 1966, and the like, and accordingly, they can be readily obtained by those skilled in the art.

The fluorescent staining agent of the present invention is uptaken by tumor cells or tumor tissues, hydrolyzed by an esterase to give a strongly fluorescent fluorescein. Types of tumor cells or tumor tissues that can be stained with the fluorescent staining agent of present invention are not particularly limited, and examples thereof include any kinds of malignant tumor cells and malignant tumor tissues such as cancer cells or cancer tissues of gastric cancer, esophagus cancer, duodenum cancer, bronchial cancer, lung cancer, colon cancer, skin cancer, and the like, but they are not limited to these examples. Although it is not intended to be bound by any specific theory, the fluorescent staining agent of the present invention is uptaken by normal cells and tumor cells in the substantially same degree, but a larger amount of fluorescein is produced in the tumor cells due to differences in intracellular esterase activity, activities of other metabolic enzymes, and the like, and/or the produced fluorescein is retained in the tumor cells for a longer period of time, resulting in stronger fluorescence staining by fluorescein in tumor cells. Therefore, the fluorescent staining agent of the present invention can be used as a staining agent selective for tumor cells or tumor tissues. Since tumor cells or tumor tissues stained with the fluorescent staining agent of the present invention emit fluorescence when irradiated with an excitation light for fluorescein, they can be easily distinguished from normal cells.

Type of the fluorescein excitation light is not particularly limited, and an excitation light having a wavelength of about 470 to 500 nm may usually be used. Although a concentration of the fluorescent staining agent to be brought into contact with tumor cells or tumor tissues is not particularly limited, the concentration may be, for example, about 1 x 10⁻⁴ to 1 x 10⁻² mg/ml. The fluorescent staining agent may be brought into contact with tumor cells or tumor tissues, then left usually at a temperature of about 37°C for several minutes to several hours, preferably about 10 minutes to 2 hours, more preferably about 30 minutes to 2 hours, to allow hydrolysis to advance, and then irradiated with an excitation light.

A diagnostic agent comprising the fluorescent staining agent of the present invention as an active ingredient can be suitably used for a purpose of cancer diagnosis, for example, early cancer diagnosis. For example, in diagnosis for a purpose of confirming the presence or absence of tumor tissues, the aforementioned diagnostic agent may be brought into contact with a site to be an object of the diagnosis (for example, skin, oral cavity, esophagus, stomach, duodenum, large intestine, bronchus, lung, and the like) by means of application, swallowing, spraying, injection, or the like, then the site may be irradiated with an excitation light several minutes to several hours, preferably about 10 minutes to 2 hours, more preferably about 30 minutes to 2 hours, after the contact, and the presence or absence of a fluorescently stained tissue may be confirmed macroscopically or microscopically.

In a more preferred embodiment, the fluorescent staining agent of the present invention can be used as a fluorescent contrast medium to perform cancer diagnosis using an endoscope. For example, the fluorescent staining agent of the present invention may be brought into contact with a site to be an object of diagnosis (for example, esophagus, stomach, duodenum, large intestine, bronchus, lung, and the like) by means of swallowing, spraying, injection, or the like, then the site may be irradiated with an excitation light from a light source of an endoscope several minutes to several hours, preferably about 10 minutes to 2 hours, more preferably about 30 minutes to 2 hours, after the contact, and the presence or absence of a fluorescently stained tissue may be endoscopically confirmed. The diagnosis can also be performed by recording an image or video in a film camera, digital camera, CCD video camera, or the like by using an endoscope. Although type of the endoscope is not particularly limited, a fluorescence endoscope that can emit an excitation light for fluorescein as a light source of an endoscope is preferred.

Further, in the aforementioned diagnostic agent, contrast medium and diagnosis method, a cyanine compound may be used with or instead of the aforementioned fluorescent staining agent. Type of the cyanine compound is not particularly limited, and any arbitrary cyanine compounds available for those skilled in the art may be used. For example, commercially available cyanine compounds such as Cy-3 and Cy-7 (both can be obtained as commercial products of Amersham Biosciences) can be preferably used. A method of performing cancer diagnosis by using a fluorescence endoscope that can emit an excitation light, in particular, for cyanine compounds as a light source of an endoscope and Cy-3 or Cy-7, preferably Cy-7, as a fluorescence contrast medium is a preferred embodiment of the present invention.

### Examples

The present invention will be explained more specifically with reference to examples. However, the scope of the present invention is not limited by the following examples.

### Example 1: Screening for candidate compounds

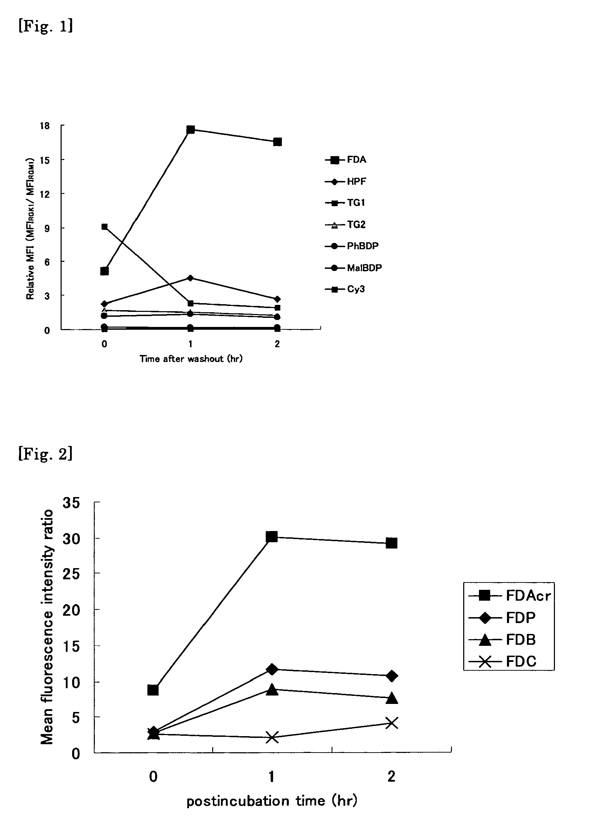

Screening for candidate compounds was performed in a cultured cell system. As cultured cells, rat normal gastric mucosal epithelium derived cells, RGM1, and transformed cells thereof, RGK1, were used as model cells of normal cell and cancer cell, respectively. For the culture of RGM1 and RGK1, a DMEM/HAM F12 (1:1) medium (10% FBS, 0.1% penicillin streptomycin) was used. Each of the compounds shown below (concentration: 0.1 µM) was loaded on these cells for 10 minutes, then the medium was replaced with the medium not containing the compounds, and after a given time, average fluorescence intensity was determined by flow cytometry. Effectiveness of each compound was evaluated on the basis of average of fluorescence intensity ratio of RGK1 relative to RGM1. As a result, fluorescein diacetate (FDA), which is a fluorescein ester derivative, gave the highest fluorescence intensity ratio, and it was found that the intensity ratio changed in a time-dependent manner (Fig. 1).

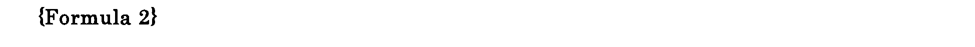

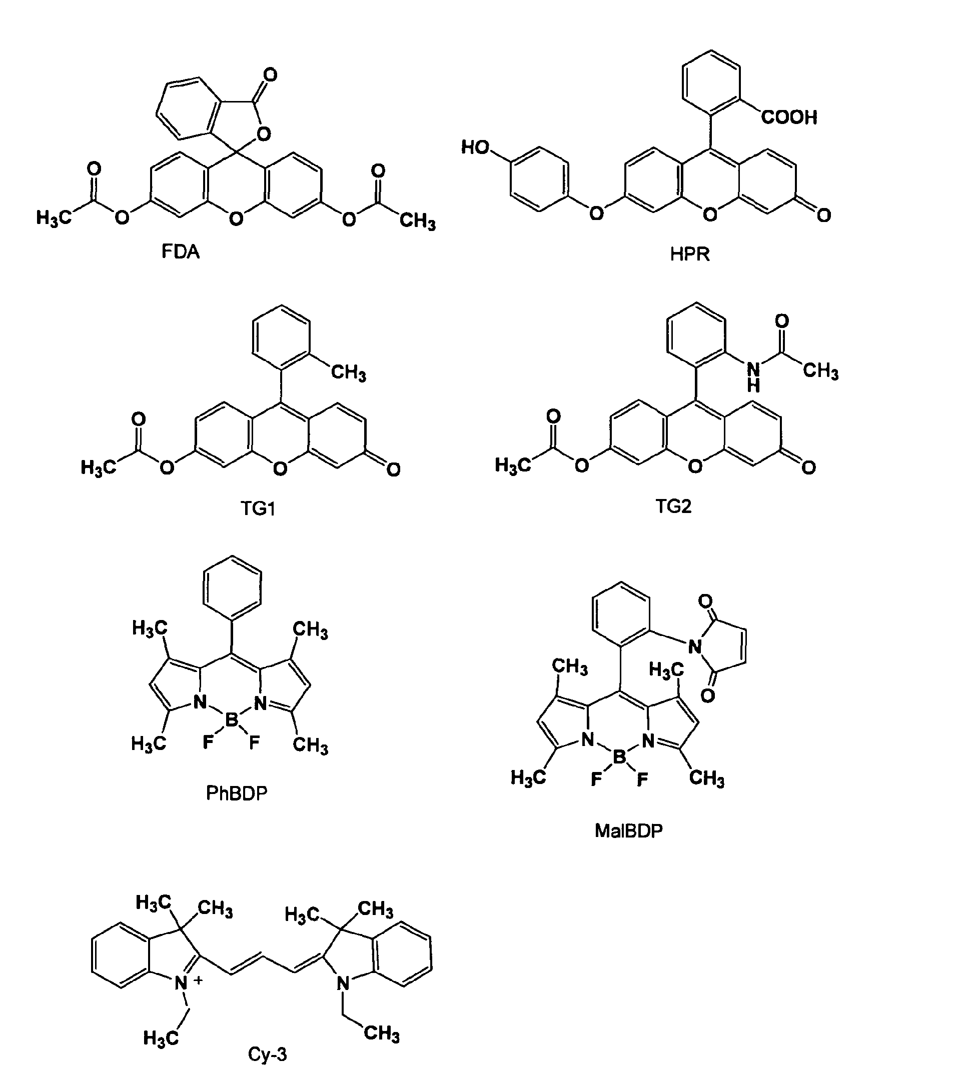

### Example 2

FDA is an esterase-sensitive fluorescent probe, and it enters into a cell by diffusion, then is hydrolyzed by an intracellular esterase to produce a fluorescent fluorescein, and gradually leaks out of the cell. Since the fluorescein is more hydrophilic compared with FDA, the leakage of the fluorescein out of the cell occurs much more slowly than the uptake of FDA into the cell. This process was compared for the normal cells and the cancer cells. When the hydrolytic enzyme activity was compared for the two types of cell lysates, it was found that RGK1 had an about twice higher hydrolytic activity for FDA. Further, when the fluorescein leakage rates were compared, it was found that leakage from RGK1 was slower than that from RGM1. On the basis of these results, it was considered that FDA provided a higher fluorescence intensity in cancer cells than in normal cells due to two of the factors, i.e., the higher hydrolytic enzyme activity and the lower leakage rate in the cancer cells.

### Example 3

Various ester derivatives converted from acetyl ester of FDA shown in Table 1 were compared by flow cytometry in the same manner as that used in Example 1. The results are shown in Fig. 2. As a result, it was found that fluorescein diacrylate (FDAcr) had a superior performance for distinguishing cancer cells from normal cells.

**[Table 1]**

| R¹ and R² | Abbreviation |
|---|---|
| -CH=CH₂ | FDAcr |
| -CH₂-CH₃ | FDP |
| -CH₂-CH₂-CH₂ | FDB |
| -CH₂-CH₂-CH₂-CH₃ | FDC |

### Example 4

Imaging was performed by using FDAcr under a fluorescence microscope. Strong fluorescence was observed in the cancer cells by observation under a fluorescence microscope (Olympus Corporation, IX71, excitation wavelength: 470-490 nm, filter: 500 nm), as in the results of Example 3, and thus it was confirmed that the cancer cells and normal cells were clearly distinguishable.

### Example 5

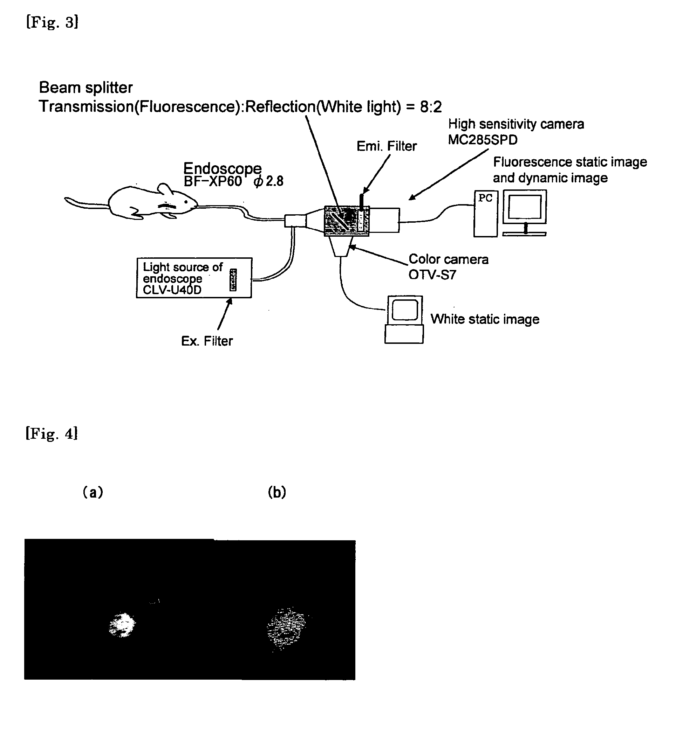

By using FDAcr, imaging of an NMBA rat, an esophagus cancer model, was performed. A rat (10-week old, male) was subcutaneously injected with 1 mg of NMBA 5 times per week for the first to 5th weeks, and once per week for 6th to 15th weeks, and used for the experiment after 16th week. The rat was anesthetized by intraperitoneal administration of Nembutal (1 mL/kg body weight) and fixed on a rest in the dorsal position to maintain the airway. An endoscope was inserted into the esophagus for observation. The experimental system is shown in Fig. 3, and the results are shown in Fig. 4. Fluorescence intensity around the esophagus cancer lesion was observed to be higher than that of normal tissues. From these results, it was demonstrated that cancer lesions and normal tissues were successfully distinguished by using FDAcr under an endoscope.

### Example 6

In the same manner as Example 5, a fluorescent endoscopic image was obtained by using Cy-7, a cyanine near-infrared fluorescent dye. When Cy-7 was used, fluorescence emitted from Cy-7 was observed mainly in a nodular lesion protruding in the esophagus, although fluorescence was also observed in portions that appeared to be non-pathological lesion, and thus it was demonstrated that the lesion was fluorescently stained.

### Example 7

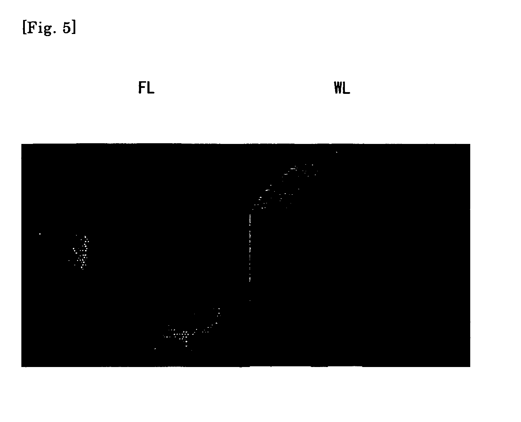

By using FEAcr, imaging of a colon cancer model rat was performed. A colon cancer model rat was anesthetized with ether, and inside the colon was washed with 0.6% aqueous acetic acid. The inside of the colon was washed two to three times with PBS maintained at 37°C, and then an enema with 15 ml of 100 µM FDAcr (PBS solution) was performed from the anus and the anus was clogged to stand for 3 minutes. After 3 minutes, the inside of the colon was washed two to three times with PBS maintained at 37°C. A fiberscope (BF-3C40) was inserted in the colon to observe pathological legions. When feces was found to be an obstacle for the observation, the inside of the colon was appropriately washed. The results are shown in Fig. 5. Strong fluorescence was observed at each colon cancer lesion (left side wall, right lower wall), and it was demonstrated that easy diagnosis of cancer was achievable.

### Industrial Applicability

The fluorescent staining agent of the present invention is useful as a staining agent for distinguishing tumor cells or tumor tissues from normal cells or normal tissues with fluorescent staining, and can be used as, for example, a tumor tissue selective contrast medium in endoscopic diagnosis.

## Claims

1. A tumor cell or tumor tissue-selective fluorescent staining agent comprising a compound represented by the following general formula (I): 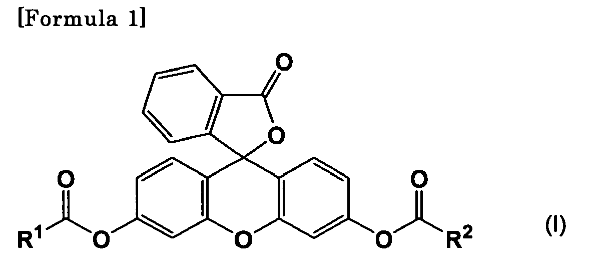 wherein R¹ and R² independently represent a C₁-C₄ alkyl group which may be substituted, a C₂-C₄ alkenyl group which may be substituted, a C₂-C₄ alkynyl group which may be substituted, an aryl group which may be substituted, or a heteroaryl group which may be substituted.

2. The fluorescent staining agent according to claim 1, wherein R¹ and R² independently represent a C₁-C₄ alkyl group, a C₂-C₄ alkenyl group, an aryl group, or a heteroaryl group.

3. The fluorescent staining agent according to claim 1, wherein R¹ and R² independently represent a C₁-C₃ alkyl group, or a C₂₋₃ alkenyl group.

4. The fluorescent staining agent according to claim 1, wherein R¹ and R² represent -CH=CH₂.

5. A diagnostic agent for cancer which comprises the fluorescent staining agent according to any one of claims 1 to 4 as an active ingredient.

6. The diagnostic agent according to claim 5, which is used for early cancer diagnosis.

7. A tumor tissue-selective contrast medium comprising the fluorescent staining agent according to any one of claims 1 to 4 as an active ingredient.

8. The contrast medium according to claim 7, which is used for endoscopic cancer diagnosis.
